# EUROPEAN PATENT APPLICATION

(11) **EP 4 066 757 A1**
(43) Date of publication of application: **05.10.2022**
(21) Application number: 22165415.5
(22) Date of filing: 30.03.2022
(51) Int. Cl.: A61B 17/29, A61B 17/16, A61B 17/295, A61B 17/00

(54) **TRIGGER DEVICE WITH ERGONOMIC HANDLE**

(30) Priority: 01.04.2021 IT 202000001676 U
(71) Applicant: Melozzi, Alessandro, 64100 Teramo (TE) (IT)
(72) Inventor: Melozzi, Alessandro, 64100 Teramo (TE) (IT)
(74) Representative: Cutropia, Gianluigi

(57) **Abstract**

A trigger device (100) comprising a body (1) with a handle (10) and a guide (11) suitable for accommodating a movable part suitable for being connected to a tool that protrudes anteriorly from the device; an actuation lever (15) pivoted to the body (1) to operate the movable part; and actuation means (L) connected to the actuation lever (15) and configured in such a way to transmit a linear translation motion to the movable part; the handle (10) and the actuation lever (15) have an ergonomic shape that fits the user's hand; in a front or rear view of the device (100), both the handle (10) and the actuation lever (15) have a curved shape with convexity facing the opposite sides with respect to an axis of symmetry (X) of the body (1) of the device.

## Description

The present invention relates to a trigger device having a handle with a trigger that pushes a movable part. Such a device may be used for a pliers having a tip with a fixed jaw and a movable jaw for clamping or cutting an object or for a rotary tool, such as a screwdriver, wherein the movement of the trigger results in the rotation of the tool. In particular, such a handle may be used for a surgical pliers for laminectomy.

EP2326262 and WO2017/072224, in the name of the same applicant, disclose a laminectomy forceps provided with a lever mechanism for multiplying the force exerted by a surgeon on an actuation lever (trigger) that determines the translation of a movable part consisting of a sliding tray that forms part of a cutting portion.

The Italian patent application No. 102019000003917, in the name of the same applicant, describes a laminectomy forceps with a rotary tip.

The trigger devices of the prior art describe a generic handle comprising a counter lever and a trigger. However, such a handle is not ergonomic and does not fit well in the user's hand.

In the case when a high force is be exerted on the trigger of the device, such in the case of surgical pliers, it is fundamental that the handle is ergonomic and fits the shape of the user's hand.

The purpose of the present invention is to overcome the drawbacks of the prior art by providing a trigger device that is efficient, effective, ergonomic, versatile, and suitable for adapting to the conformation of the user's hand.

A further purpose is to provide such a trigger device that is reliable, economical and simple and easy to use.

These purposes are achieved in accordance with the invention with the features listed in the independent claim 1.

Advantageous embodiments of the invention appear from the dependent claims.

The trigger device according to the invention is defined by claim 1.

For the sake of explanatory clarity, the description of the trigger device according to the invention is continued with reference to the attached drawings, which only have an illustrative and non-limiting value, wherein:

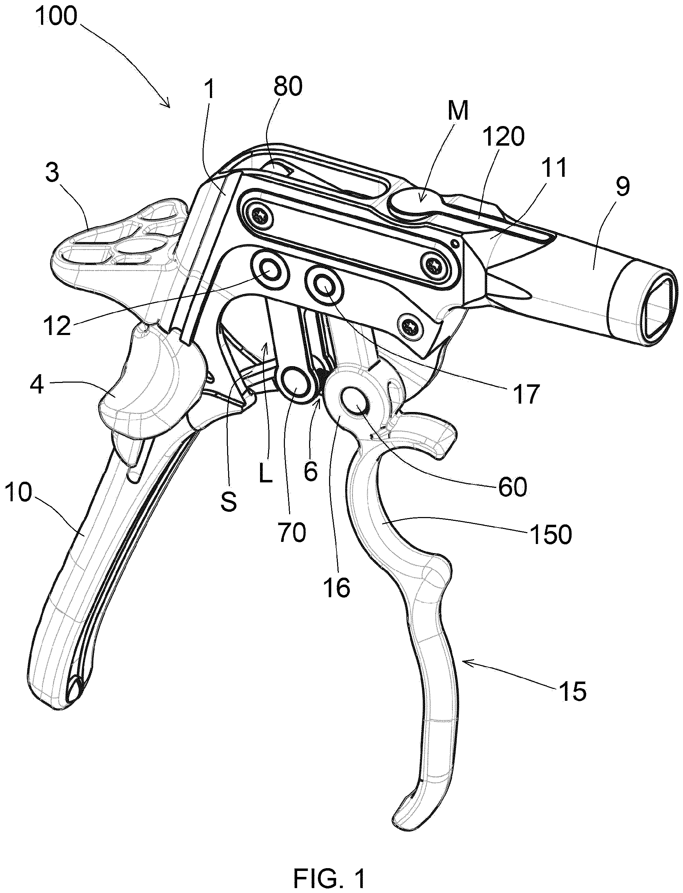

Fig. 1 is an axonometric view of a trigger device according to the invention;
Fig. 2 is an exploded view of the device in Fig. 1;

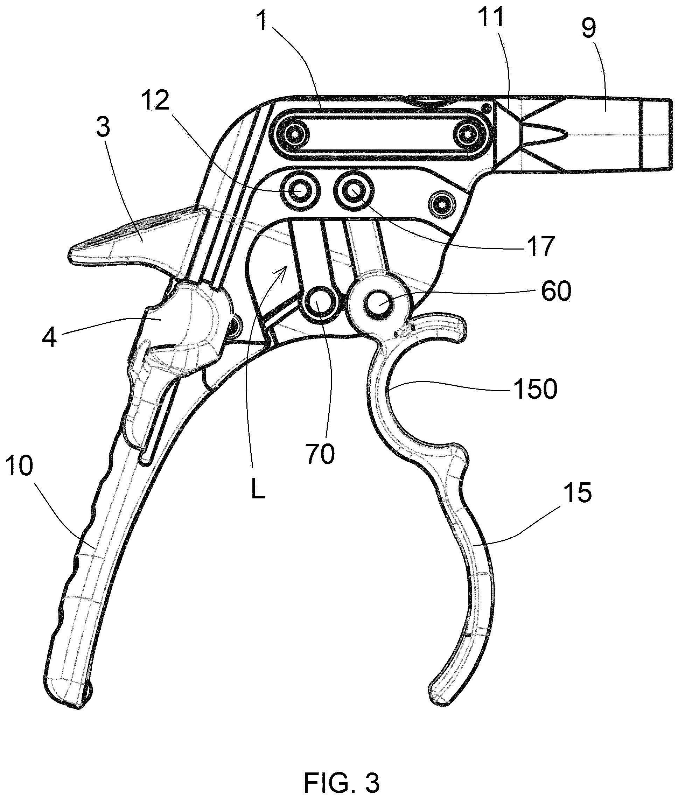

Fig. 3 is a side view of the device in Fig. 1;
Fig. 4 is a front view of the device in Fig. 1;
Fig. 5 is a rear view of the device in Fig. 1;

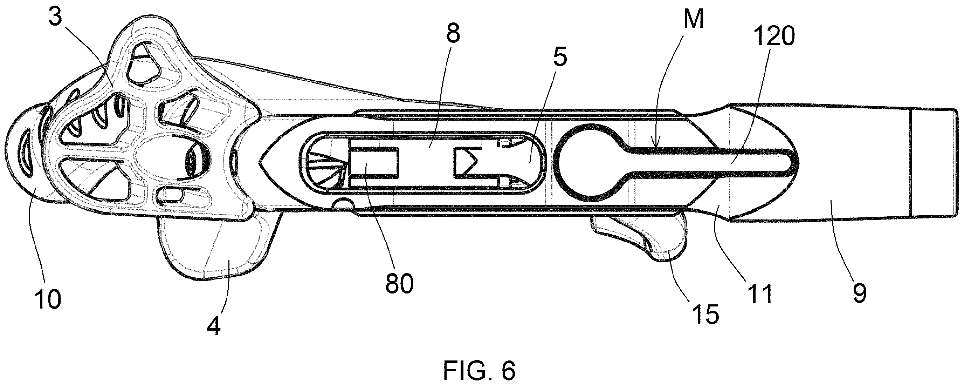

Fig. 6 is a top view of the device in Fig. 1;
Fig. 7 is an enlarged sectional view of a detail of the device, illustrating the connection and adjustment means;
Figs. 8 and 9 are two side views of the device in a minimum and maximum size configuration, respectively.

With the aid of the figures, the device according to the invention is described, it being indicated with reference numeral 100.

The device (100) comprises an L-shaped body (1) having a handle (10) and a guide (11) that protrudes orthogonally from the handle. The guide (11) may be in the form of a cylindrical shank.

A spur (3) is mounted on the handle (10) so as to project posteriorly from the handle to match an edge of the hand between the thumb and the forefinger.

A thumb support (4) is mounted on the handle, under the spur (3) projecting laterally in such a way to act as a support for the user's thumb.

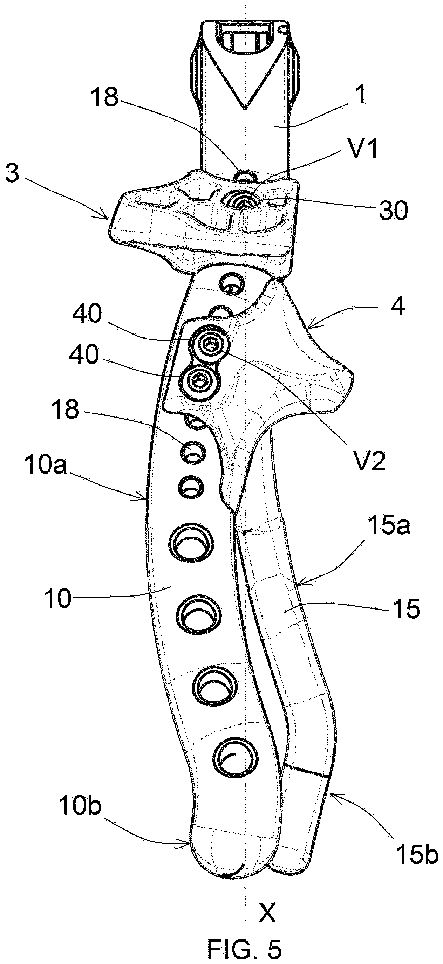

With reference to Fig. 5, the spur (3) and the thumb support (4) are adjustable in position on the handle (10) according to the size of the user's hand. For this purpose, the handle (10) comprises a plurality of fixing holes (18). The spur (3) and the thumb support (4) comprise respective holes (30, 40) suitable for accommodating screw means (V1, V2) that are engaged in the fixing holes (18) of the handle.

An actuation lever (trigger) (15) is pivoted to the body (1) and is connected to actuation means (L) suitable for transforming the movement of the actuation lever (15) into a linear translation. The actuation means (L) are suitable for being connected to a movable part (not shown) that protrudes from the guide (11).

The movable part may have a cutting tip, such as for cutting a bone, a gripping tip, or a driving tip, such as a screwdriver tip or the like.

The movable part may translate, rotate, or roto-translate. In the case of rotation and/or roto-translation, the device (100) comprise a helical coupling that transforms the linear translation into a rotation or roto-translation of the movable part.

The device has an attachment (9) wherein the movable part of the device is arranged. The attachment (9) is mounted in the guide (11) of the body. The attachment (9) comprises a plurality of radial holes (91) circumferentially disposed and angularly equidistant from each other.

Locking means (M) are mounted in the body (1) and are operated by the user to lock the attachment (9) and the movable part.

The locking means (M) comprise a locking lever (120) pivoted to the body (1) at a pivot point (121). The locking lever (120) has a ratchet (122) disposed at a front end that is engaged in a hole (91) of the attachment (9).

The actuation lever (15) is disposed in opposite position relative to the handle (10). The user places the handle (10) in the palm of his/her hand and operates the actuation lever (15) with the fingers of the same hand.

The actuation lever (15) comprises a first free end and a second end rotating about a pin (17) connected to the body (1). The actuation lever (15) comprises a rigid wing (16) facing the handle (10) for the connection with the actuation means (L).

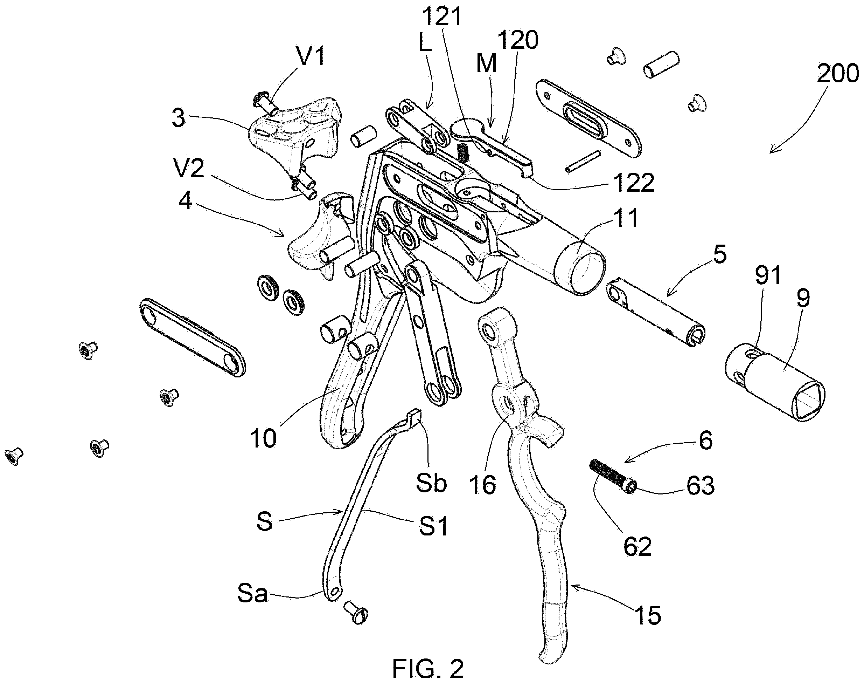

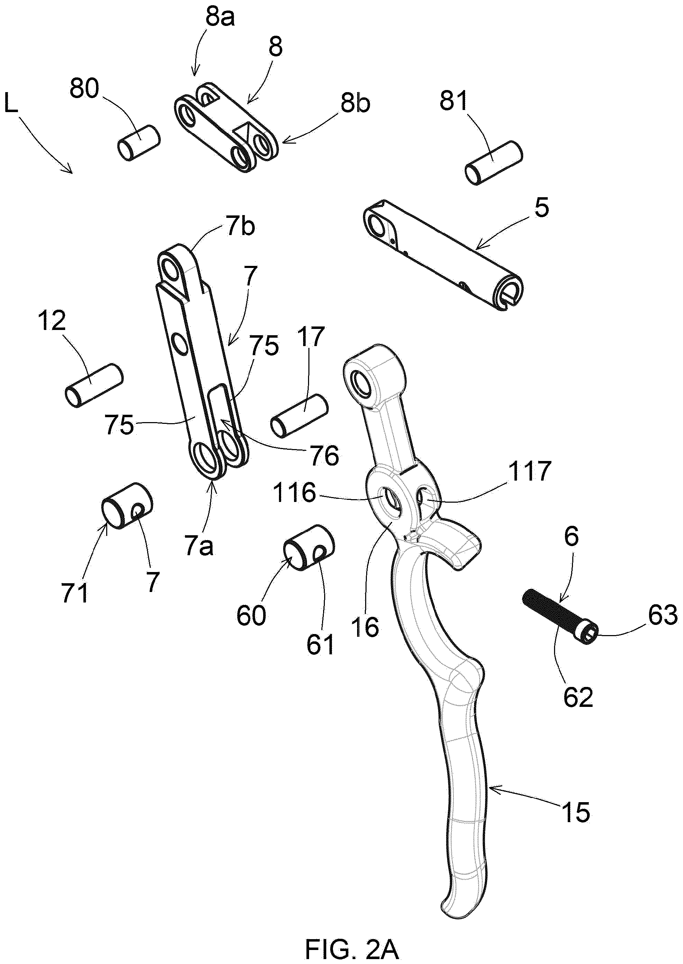

With reference to Fig. 2A, the actuation means (L) comprise a force-multiplying lever mechanism comprising two levers:
- a first lever (7) pivoted to the body (1), and
- a second lever (8) pivoted to the first lever (7).

Connection means (6) connect the rigid wing (16) of the actuation lever to the first lever (7).

The rigid wing (16) is in the form of a bushing or flange having a hole (116) wherein a first pivot pin (60) with a radial hole (61) is disposed. The wing (16) has a radial slot (117) that crosses the hole (116) of the wing.

The first lever (7) is a straight bar, rotatably mounted about a fulcrum (12) attached to the body (1), so as to define two arms. The first lever (7) comprises:
- a first arm between the fulcrum (12) and a first end (7a) of the first lever and
- a second arm between the fulcrum (12) and a second end (7b) of the first lever.

A second pivot pin (70) having a threaded radial hole (71) is arranged in the first end (7a) of the first lever.

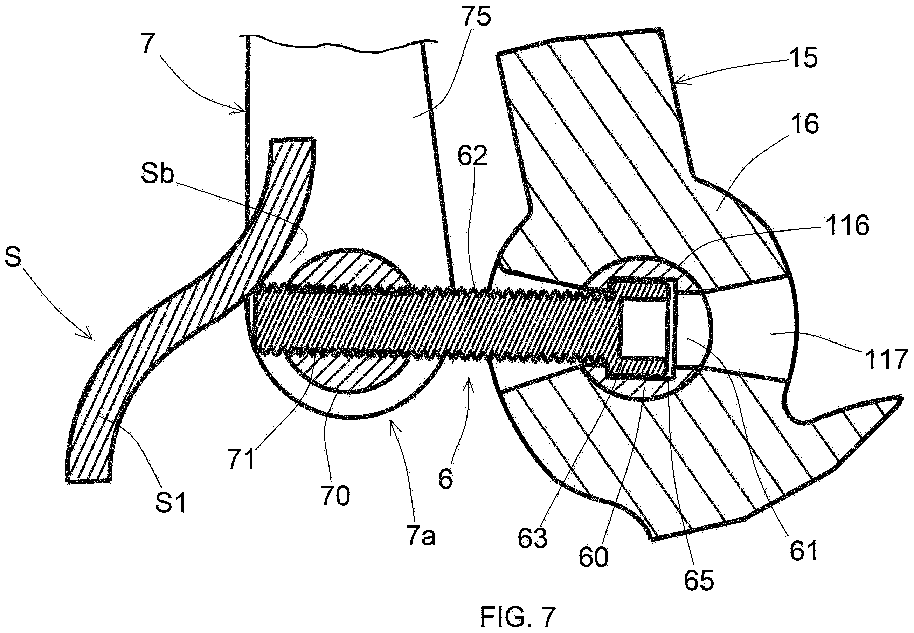

With reference also to Fig. 7, the connection means (6) comprise an adjusting screw (62) with a head (63).

The radial hole (61) of the first pivot (60) has a seat (65) suitable for accommodating the head (63) of the screw.

The adjusting screw (62) is inserted in the radial slot (117) of the rigid wing of the actuation lever, in the radial hole (61) of the first pivot pin (60) and is screwed into the radial hole of the second pivot pin (70), until the head (63) of the screw is accommodated in the seat (65) of the first pivot pin (60).

In view of the above, a movement of the actuation lever (15) moves the connection means (6) which in turn move the first lever (7).

In addition, the connection means (6) act as adjustment means to adjust the distance between the handle (10) and the actuation lever (15) according to the size of the user's hand.

By tightening the adjustment screw (62), the distance between the handle (10) and the actuation lever (15) is reduced, whereas by unscrewing the adjustment screw (62) the distance between the handle (10) and the actuation lever (15) is increased.

The first arm of the first lever (7) is longer than the second arm. The first lever (7) is longer than the adjustment screw (62). The second lever (8) is shaped as a straight bar with shorter length than the first lever (7).

The second lever (8) comprises a first end (8a) pivoted to the first lever (7) by means of a third pin (80), and a second end (8b) pivoted by means of a fourth pin (81) to a connection member (5), in the form of a sleeve, which is slidingly mounted in the guide (11) of the body. The first lever (7) of the force multiplying lever mechanism acts as link block and the second lever (8) acts as connecting rod.

Although in the Figures the actuation means (L) consist in a specific force multiplying lever mechanism, such actuation means (L) may comprise a different lever mechanism or even an actuator suitable for imparting a linear motion of the connection member (5) when the actuation lever (15) is moved.

With reference to Fig. 2, automatic return means (S) are suitable for holding the actuation lever (15) in open position. The automatic return means (S) comprise a leaf spring (S1) mounted in the handle (10) and facing the actuation lever. The spring (S1) has a first end (Sa) attached to the handle and a second end (Sb) protruding from the handle to butt against an end of the adjusting screw (62) of the connection means that protrudes from the second pivot pin (70).

The first end (7a) of the first lever (7) is shaped like a fork with two plates (75) parallel to each other which generate a gap (76) wherein the second end (Sb) of the spring (S1) is fitted.

The handle (10) and the actuation lever (15) are ergonomically shaped to fit the user's hand.

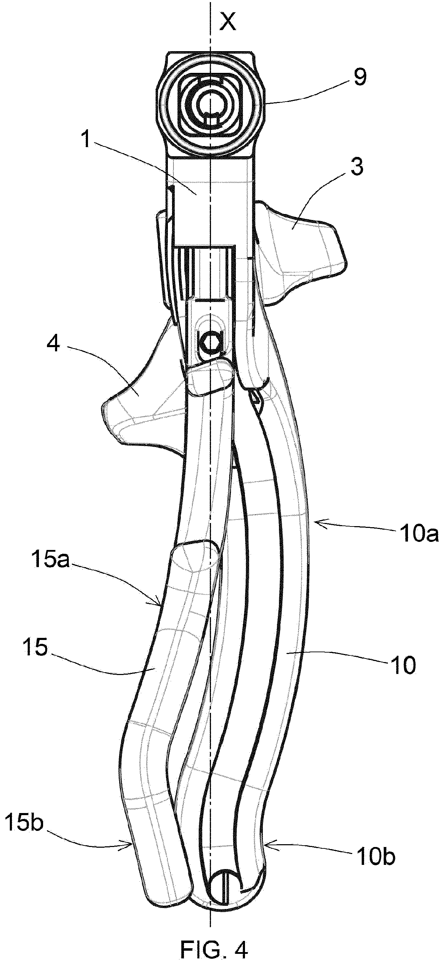

With reference to Figs. 4 and 5, in a front or rear view of the device (100), both the handle (10) and the actuation lever (15) have a curved shape with convexity on opposite sides with respect to an axis of symmetry (X) of the body (1) of the device.

The Figures show a device for left-handed users, wherein, with reference to the rear view of Fig. 5, the handle (10) has a leftward convexity and the actuation lever (15) has a rightward convexity. For a device for right-handed users, the convexities of the handle (10) and of the actuation lever (15) are reversed.

The handle (10) has a curved portion (10a) that terminates in a substantially straight lower portion (10b).

The actuation lever (15) has an upper portion (15a) in divergent position with respect to the axis of symmetry (X) and a lower portion (15b) in convergent position with respect to the axis of symmetry (X).

The actuation lever (15) has a "C"-shaped forefinger seat (150) in side view, located under the rigid wing (16) and suitable for accommodating the forefinger of the user's hand.

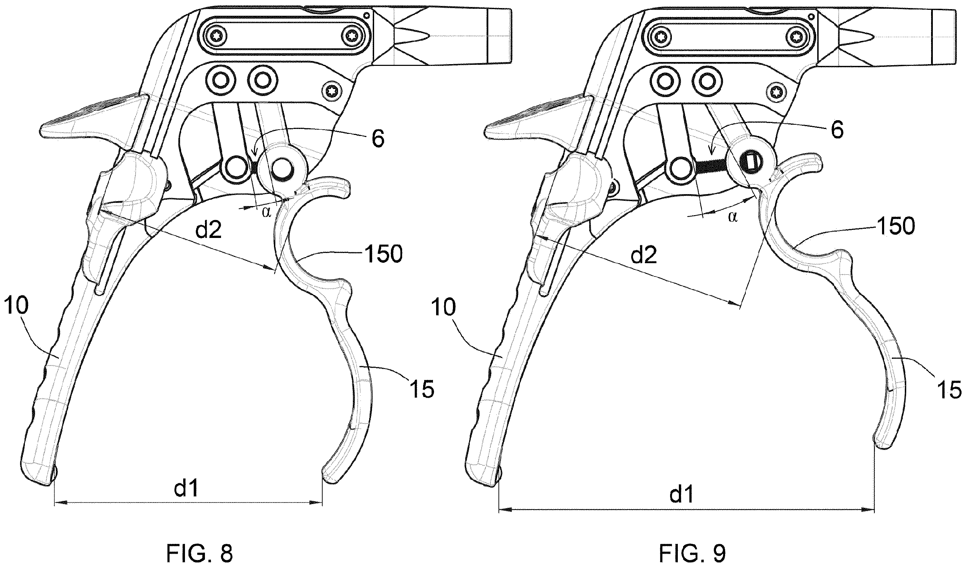

Figs. 8 and 9 illustrate:
- a first distance (d1) between the lower ends of the handle (10) and the actuation lever (15),
- a second distance (d2) between the forefinger seat (150) of the actuation lever (15) and the handle (10),
- an angle (α ) between the handle (10) and the actuation lever (15).

Due to the fact that the connection means (6) operate as adjustment means, the first distance (d1), the second distance (d2) and the angle (α) between the handle (10) and the actuation lever (15) can be adjusted. By way of example, the angle (α) can vary from 2° to 20°, the first distance d1 can vary from 70 mm to 110 mm, and the second distance d2 can vary from 48 mm to 65 mm.

## Claims

1. A trigger device (100) comprising:
- a body (1) having a handle (10) and a guide (11) suitable for accommodating a movable part suitable for being connected to a tool that protrudes anteriorly from the device;
- an actuation lever (15) pivoted to the body (1) to operate the movable part; and
- actuation means (L) connected to the actuation lever (15) and configured in such a way to transmit a linear translation motion to the movable part;
**characterized by** the fact that
said handle (10) and said actuation lever (15) are ergonomically shaped to fit the user's hand,
wherein, in a front or rear view of the device (100), both the handle (10) and the actuation lever (15) have a curved shape with convexity on opposite sides with respect to an axis of symmetry (X) of the body (1) of the device.

2. The device (100) according to claim 1, wherein the handle (10) has a curved portion (10a) terminating in a substantially straight lower portion (10b).

3. The device (100) according to claim 1 or 2, wherein the actuation lever (15) has an upper portion (15a) in divergent position with respect to the axis of symmetry (X) of the body and a lower portion (15b) in convergent position with respect to the axis of symmetry (X).

4. The device (100) according to any one of the preceding claims, wherein the actuation lever (15) has a "C"-shaped forefinger seat (150) in side view, suitable for accommodating the forefinger of the user's hand.

5. The device (100) according to any one of the preceding claims, wherein.
the actuation lever (15) has a rigid wing (16) in the form of a bushing or flange,
the actuation means (L) comprise a first lever (7), and
said device (100) comprises connection means (6) to connect the rigid wing (16) of the actuation lever to the first lever (7) of the actuation means (L), wherein said connection means (6) also operate as adjustment means for adjusting the distance between the handle (10) and the actuation lever (15) according to the size of the user's hand.

6. The device (100) according to claim 5, wherein said connection means (6) comprises an adjusting screw (62).

7. The device (100) according to claim 6, wherein.
said rigid wing (16) of the actuation lever has a hole (116) wherein a first pivot pin (60) with a radial hole (61) is disposed, and said rigid wing (16) has a radial slot (117) that crosse the hole (116) of the rigid wing;
a second pivot pin (70) with a threaded radial hole (71) is disposed in a first end (7a) of the first lever of the actuation means; and
said adjusting screw (62) is inserted in the radial slot (117) of the rigid wing of the actuation lever, in the radial hole (61) of the first pivot pin (60) and is screwed into the radial hole (71) of the second pivot pin (70).

8. The device (100) according to claim 7, wherein said adjusting screw (62) has a head (63) and said radial hole (61) of the first pivot pin (60) has a seat (65) suitable for accommodating the head (63) of the adjusting screw.

9. The device (100) according to any one of the preceding claims, further comprising:
- a spur (3) mounted on the handle (10) so as to project posteriorly from the handle to match with an edge of the hand between the thumb and the forefinger, and
- a thumb support (4) mounted on the handle, under the spur (3), projecting laterally in such a way to act as a support for the user's thumb.

10. The device (100) according to claim 9, wherein said spur (3) and said thumb support (4) are adjustable in position on the handle (10) according to the size of the user's hand.

11. The device (100) according to claim 10, wherein the handle (10) comprises a plurality of fixing holes (18) and the spur (3) and the thumb support (4) comprise respective holes (30, 40) suitable for accommodating screw means (V1, V2) that are engaged in the fixing holes (18) of the handle.

12. The device (100) according to any one of the preceding claims, further comprising automatic return means (S) for maintaining the actuation lever (15) in open position, wherein said automatic return means (S) comprise a leaf spring (S1) mounted in the handle (10) and facing the actuation lever (15).

13. The device (100) according to claim 12, when dependent on any one of claims 6 to 11, wherein said spring (S1) has a first end (Sa) fixed to the handle (10) and a second end (Sb) protruding from the handle to butt against an end of the adjusting screw (62) of the connection means protruding from the second pivot pin (70).

14. The device (100) according to claim 13, wherein the first end (7a) of the first lever (7) of the actuation means is shaped like a fork with two plates (75) parallel to each other which generate a gap (76) wherein the second end (Sb) of the spring (S1) is inserted.
